# EUROPÄISCHE PATENTANMELDUNG

(11) **EP 1 611 907 A1**
(43) Veröffentlichungstag der Anmeldung: **04.01.2006**
(21) Anmeldenummer: 04024326.3
(22) Anmeldetag: 13.10.2004
(51) Int. Cl.: A61M 1/36, A61M 1/34, B01D 29/58

(54) **Blutaufbereitungsanlage**

(30) Priorität: 29.06.2004 DE 102004031544
(71) Anmelder: Herbener, Heinz-Gerd, 52249 Eschweiler (DE); Backes, Herbert, 53424 Remagen (DE); Latza, Reinhard, Dr. med., 66386 St. Ingbert (DE); Borowsky, Richard, Dipl.-Kfm., 52249 Eschweiler (DE)
(72) Erfinder: Backes, Herbert, 53424 Remagen (DE)
(74) Vertreter: Patentanwaltskanzlei Liermann-Castell

(57) **Zusammenfassung**

Es ist bekannt, Blut - insbesondere von Menschen - in die einzelnen Bestandteile zu zerlegen, um bei der Behandlung eines Patienten dem Patienten genau die Blutbestandteile zuzuführen, die notwendig sind. Hierzu wird das Blut üblicherweise in die Bestandteile Leukozyten, Erythrozyten, Thrombozyten und Serum zerlegt.

Derartige Blutaufbereitungsanlagen sind jedoch aufwändig in der Herstellung und im Gebrauch. Es ist daher insbesondere in unterentwickelten Ländern häufig nicht möglich, das entsprechende Equipment für eine ordnungsgemäße Blutaufbereitung zur Verfügung zu stellen. Der Erfindung liegt daher die Aufgabe zugrunde, eine Blutaufbereitungsanlage bereitzustellen, die einfach herstellbar und nutzbar ist. Diese Aufgabe wird durch eine Blutaufbereitungsanlage mit einem Gehäuse gelöst, welche einen Einlass und mehrere Auslässe aufweist, wobei der Einlass und die Auslässe über Filter miteinander in Verbindung stehen. Der Erfindung liegt die Erkenntnis zugrunde, dass in einem einfachen handlichen Gehäuse die gesamte Blutaufbereitungsanlage untergebracht werden kann.

## Beschreibung

Bluttransfusionen sind absolut lebensnotwendig für Millionen von Patienten und müssen weltweit täglich akut oder routinemäßig durchgeführt werden. Da eine moderne Transfusion nach Maß verlangt wird dürfen in modemen Ländern nur Blutkomponente (Retrozytenkonzentrate, Thrombozytenkonzentrate und gefrorenes Frischplasma) und kein Vollblut verwendet werden. Die Komponenten werden maschinell im Rahmen einer Mulikomponentenspende oder selektiv von einem geeigneten Blutspender gewonnen. Erythrozytenkonzentrate werden vorwiegend aus frisch abgenommenem Vollblut gewonnen. Die Spende von Vollblut (450 ml oder 500 ml) erfolgt innerhalb von 10 Minuten in einem Primärbeutel, der 63 ml bzw. 70 ml CPD (Citrat, Phosphat, Dextrose) und CPD mit Zusatz von Adenin (CPDA-1) enthält. Das Blut muss vor Lagerung leukozytendepletiert und in Einzelkomponenten getrennt werden (Erythrozytenkonzentrate und ggf. Thrombozyten). Für die Leukozytendepletion werden geeignete Leukozytenfilter und zur Auftrennung von Vollblut werden spezielle Zentrifugen und Bluttrenngeräte verwendet. Arbeitsmethoden sind häufig ungenau und müssen deshalb anhand von Stichproben kontrolliert werden. Die Anforderungen an die Produktqualität sind in nationalen und europäischen Gesetzen und Richtlinien vorgegeben (Guide to the preparation, use and quality of blood components, Council of Europe Publishing) in der jeweils gültigen Fassung; Richtlinien zur Gewinnung von Blut und Blutbestandteilen und zur Anwendung von Blutprodukten (Hämotherapie), aufgestellt vom Wissenschaftlichen Beirat der Bundesärztekammer und vom Paul Ehrlich Institut, Dt. Ärzte-Verlag, in der jeweils gültigen Fassung.

Es ist bekannt, Blut - insbesondere von Menschen - in die einzelnen Bestandteile zu zerlegen, um bei der Behandlung eines Patienten dem Patienten genau die Blutbestandteile zuzuführen, die notwendig sind. Hierzu wird das Blut üblicherweise in die Bestandteile Leukozyten, Erythrozyten, Thrombozyten und Serum zerlegt.

Derartige Blutaufbereitungsanlagen sind jedoch aufwändig in der Herstellung und im Gebrauch. Es ist daher insbesondere in unterentwickelten Ländern häufig nicht möglich, das entsprechende Equipment für eine ordnungsgemäße Blutaufbereitung zur Verfügung zu stellen.

Der Erfindung liegt daher die Aufgabe zugrunde, eine Blutaufbereitungsanlage bereitzustellen, die einfach herstellbar und nutzbar ist.

Diese Aufgabe wird mit einer Blutaufbereitungsanlage gelöst, die ein Gehäuse mit einem Einlass und mehreren Auslässen aufweist, wobei der Einlass und die Auslässe über Filter miteinander in Verbindung stehen.

Der Erfindung liegt die Erkenntnis zugrunde, dass in einem einfachen handlichen Gehäuse die gesamte Blutaufbereitungsanlage untergebracht werden kann. Ein derartiger Blutbestandteilseparierer nutzt zur Trennung der Blutbestandteile Filter unterschiedlicher Porositäten und ein Verstopfen der Filter oder eine Verschmutzung der Oberflächen wird durch eine super-hydrophobe Oberfläche vermieden. Die Oberfläche ist hierbei derart hydrophob, dass sich die Blutbestandteile an den Oberflächen nicht anlegen sondern von den Oberflächen abgestoßen werden.

Ein bevorzugtes Ausführungsbeispiel sieht vor, dass ein erster Filter eine Porosität von ca. 150 µm aufweist. Dieser Filter ist vorzugsweise direkt hinter dem Einlass angeordnet und dient dazu größere Blutbestandteile wie insbesondere auch Verschmutzungen fernzuhalten. Leukozyten, Erythrozyten, Thrombozyten und das Serum können diesen Filter passieren.

Hinter dem ersten Filter ist vorzugsweise ein zweiter Filter mit einer Porosität von ca. 10 µm angeordnet. Dieser Filter sorgt dafür, dass die Leukozyten zurückgehalten werden, um sie vor dem Filter aus dem Gehäuse zu entnehmen.

Hinter diesem zweiten Filter ist vorzugsweise ein dritter Filter angeordnet, der eine Porosität von ca. 3 µm aufweist. Dieser Filter lässt die Thrombozyten passieren, während die Erythrozyten zurückgehalten werden, um sie an einem weiteren Auslass aus dem Gehäuse zu entnehmen.

Die Filter bilden zwischen dem Einlass und dem Auslass für die Thrombozyten mehrere hintereinander angeordnete Kammern. Diese Kammern stehen vorzugsweise mit einem vierten Filter in Verbindung, der eine Porosität von ca. 1,5 bis 2 µm aufweist. Dieser vierte Filter ermöglicht es, aus den einzelnen Kammern das Blutserum zu entfernen und einem weiteren Auslass zuzuführen.

Die vorliegenden Entwicklung erleichtert die Auftrennung und ermöglicht sie am Ort der Blutspende bzw. am Krankenbett. Das Einwegsystem besteht aus einer doppelhalsigen "Flasche" mit mehreren Ein- und Auslassstellen. Eine mikrostrukturierte, superhydrophobe Filterplatte mit Poren, die kleiner sind als die kleinsten Blutzellen, teilt die "Flasche" vertikal in zwei Teile. Diese Filterplatte trennt Serum von den übrigen Blutbestandteilen ab und sammelt es im unteren Teil, wo es am Auslass Serum entnommen werden kann (siehe Zeichnung). Der obere Teil der Flasche wird durch drei vertikale Filter mit unterschiedlicher Porengröße in vier Kompartimente unterteilt. Diese Filter sind wie die Innenwand des oberen Teils ebenfalls superhydrophob beschichtet und somit ein Verstopfen der Poren verhindert. Im ersten, hinteren Kompartiment befindet sich die Einlassstelle für das Vollblut, das mit einem Druck p in den Blutbestandteilseparator eingeleitet wird. Das erste Filter mit einer relativ großen Porengröße dient dazu Agglutinate (Gerinsel) zurückzuhalten. Das zweite Filter mit einer Porengröße von 10-15 µ trennt Leukozyten ab. Die dritte Filterscheibe mit einer Porengröße von ca. 3 µ separiert schließlich Erythrozyten von Thrombozyten. Die Thrombozyten werden an der Auslassstelle des oberen Flaschenhalses entnommen. Um die Zellbestandteile resuspendieren zu können, müssen durch dafür vorgesehene Einlassstellen Resuspensionsflüssigkeiten zugegeben werden. Die Auslassstellen für Leukozyten und Erythrozyten befinden sich auf dem Deckel der Flasche (ähnlich der Einlassstelle für das Vollblut). Durch Verkippen der Flasche um 90 - 180° wird so ein Vermischen des Resuspensionsmedien verhindert. Eine einfache Vorrichtung ermöglicht es die "Flasche" je nach Bedarf zu kippen, durch Vibrationen oder Ultraschall die Zellen zu resuspendieren und schließlich zu definierten Behältnissen zu leiten.

In vielen Fällen genügt es, Serum von Erythrozyten und Thrombozyten bzw. Serum von Erythrozyten und diese von Thrombozyten zu trennen. Agglutinate und Leukozyten werden dabei durch vorgeschaltete Filtersysteme aus dem Blut entfernt. Für solche Fragestellungen lässt sich das oben beschriebene System entsprechend vereinfachen. Doch spielen auch hierbei die mikrostrukturierten Sieb- und Filterplatten mit ihren oben beschriebenen Oberflächeneigenschaften für die Funktion als Blutbestandteilseparator eine entscheidende Rolle.

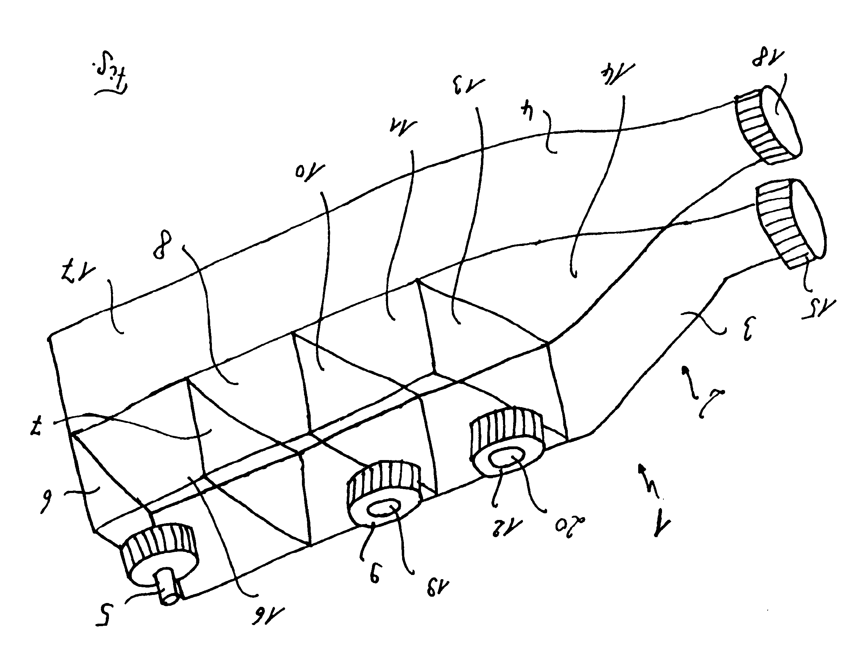

Die beiliegende Figur zeigt die auch als Blutbestandteilseparierer bezeichnete Blutaufbereitungsanlage 1 mit ihrem Gehäuse 2, das aus zwei miteinander verbundenen flaschenartigen Elementen 3 und 4 besteht. Das erste flaschenartige Element 3 weist einen Einlass 5 auf, der mit einer ersten Kammer 6 der ersten Flasche 3 in Verbindung steht. Diese Kammer 6 weist einen Filter 7 auf, der die Kammer 6 mit einer weiteren Kammer 8 verbindet. Die Kammer 8 hat einen Auslass 9 und dem Filter 7 gegenüberliegend einen zweiten Filter 10. Dieser zweite Filter 10 verbindet die Kammer 8 mit einer dritten Kammer 11. Diese dritte Kammer 11 weist wiederum einen Auslass 12 auf, um Blutbestandteile aus dem Gehäuse 2 zu entnehmen. Dem zweiten Filter 10 gegenüberliegend weist die Kammer 11 einen dritten Filter 13 auf, der die Kammer 11 mit einer im Flaschenhals angeordneten Kammer 14 verbindet. Diese Kammer 14 weist einen Auslass 15 auf, über den weitere Blutbestandteile aus dem System entnommen werden können.

Die Kammern 6, 8 und 11 und 14 stehen über einen vierten Filter 16 mit einem Gehäuse 17 in Verbindung. Dieses Gehäuse 17 ist unterhalb den bisher beschriebenen Gehäusen angeordnet und dient dazu das Blutserum zu sammeln, um es am Auslass 18 aus dem Gehäuse zu entnehmen.

Zur Verwendung der Vorrichtung wird mit Druck Blut am Einlass 5 in die erste Kammer 6 gefüllt. Blutserum gelangt durch den Filter 16 in die Kammer 17, größere Blutbestandteile verbleiben in der Kammer, und alle weiteren Blutbestandteile gelangen durch den Filter 7 in einer Größe von etwa 150 µm zunächst in die Kammer 8. Dort können am Auslass 9 Leukozyten entnommen werden. Hierzu ist im Auslass 9 ein Septum 19 vorgesehen.

Weitere Blutbestandteile gelangen durch den zweiten Filter 10 in die Kammer 11, in der Erythrozyten am Auslass 12 über ein Septum 20 entnommen werden. Über den dritten Filter gelangen Thrombozyten zum Auslass 15. Alle Kammern sind über einen vierten Filter mit einer Porosität von 1,5 bis 2 µm mit der Kammer 17 verbunden, in der sich Serum sammelt, welches über den Auslass 18 aus dem Gehäuse entnehmbar ist.

Die inneren Oberflächen des Gehäuses sowie die Filter sind extrem hydrophob ausgebildet, damit sich die Blutbestandteile nicht an den Oberflächen anlegen.

## Patentansprüche

1. Blutaufbereitungsanlage mit einem Gehäuse, das einen Einlass und mehrere Auslässe aufweist, wobei der Einlass und die Auslässe über Filter miteinander in Verbindung stehen.

2. Blutaufbereitungsanlage nach Anspruch 1, ***dadurch gekennzeichnet, dass*** die Oberflächen zwischen dem Einlass und dem Auslass, insbesondere die Filter super-hydrophob ausgeführt sind.

3. Blutaufbereitungsanlage nach Anspruch 1 oder 2, ***dadurch gekennzeichnet, dass*** ein erster Filter eine Porosität von ca. 150 µm aufweist.

4. Blutaufbereitungsanlage nach einem der vorhergehenden Ansprüche, ***dadurch gekennzeichnet, dass*** ein zweiter Filter eine Porosität von ca. 10 µm aufweist.

5. Blutaufbereitungsanlage nach einem der vorhergehenden Ansprüche, ***dadurch gekennzeichnet, dass*** ein dritter Filter eine Porosität von ca. 3 µm aufweist.

6. Blutaufbereitungsanlage nach einem der vorhergehenden Ansprüche, ***dadurch gekennzeichnet, dass*** ein vierter Filter eine Porosität von ca. 1,5 und 2 µm aufweist.
